# EUROPEAN PATENT APPLICATION

(11) **EP 2 075 610 A1**
(43) Date of publication of application: **01.07.2009**
(21) Application number: 07740087.7
(22) Date of filing: 28.03.2007
(51) Int. Cl.: G02B 7/02, A61B 1/04

(54) **IMAGING UNIT AND ENDOSCOPE USING IT**

(30) Priority: 20.09.2006 JP 2006254819
(71) Applicant: Olympus Medical Systems Corp., Tokyo 151-0072 (JP)
(72) Inventor: ICHIMURA, Hironobu, Tokyo 151-0072 (JP); TANII, Yoshiyuki, Shibuya-ku Tokyo 151-0072 (JP); ABE, Makoto, Shibuya-ku Tokyo 151-0072 (JP); ISHIZAKI, Ryosuke, Shibuya-ku Tokyo 151-0072 (JP)
(74) Representative: von Hellfeld, Axel
(86) International application number: PCT/JP2007/056649
(87) International publication number: WO 2008/035470

(57) **Abstract**

According to this invention, in order to provide an image pickup unit with an objective lens unit using a plastic lens which contributes to an improvement in assemblability and has easily achieved a reduction in optical variations by taking member properties into consideration and an endoscope using the image pickup unit, the image pickup unit is configured to include an objective lens unit composed of a plurality of optical members arranged in series with each other in a direction parallel to a single optical axis such that optical axes of the plurality of optical members coincide with each other and a series-connecting portion which unitizes the plurality of optical members and a photoelectric conversion image pickup device which receives an optical image formed by the objective lens unit and performs photoelectric conversion processing, and the series-connecting portion is configured to include a holding portion which holds one of the plurality of optical members, an extending portion which is provided to extend from one end of the holding portion in a direction parallel to the optical axis of the optical member held by the holding portion and is formed to be elastically deformable in a direction almost orthogonal to the optical axis, and an engaging portion which is provided at the extending portion and engages with, of the plurality of optical members, one different from the optical member held by the holding portion.

## Description

### Technical Field

The present invention relates to an image pickup unit and an endoscope using the image pickup unit and, more particularly, to an image pickup unit including an objective lens unit composed of a plurality of optical members and a photoelectric conversion device which photoelectrically converts an optical image formed by the objective lens unit and an endoscope using the image pickup unit.

### Background Art

An image pickup unit including an objective lens unit composed of a plurality of optical members and a photoelectric conversion device such as an image pickup device which photoelectrically converts an optical image formed by the objective lens unit is widely in practical use. Such image pickup units are used in pieces of electronic equipment such as an electronic endoscope, digital camera, and cellular phone and have come into wide use.

Examples of an optical member used in an objective lens unit of a conventional image pickup unit include a plastic lens formed of a member made of a transparent resin such as plastic, in addition to a general glass lens. In particular, a plastic lens can be formed in a desired form by molding or the like and thus has the advantage of a high degree of flexibility in shape.

Accordingly, use of a plastic lens is advantageous, e.g., in that the number of members can be reduced by inventively designing a shape of each member at the time of formation of an objective lens unit obtained by fixing a plurality of optical members in a predetermined arrangement and unitizing the plurality of optical members.

However, an objective lens unit of an image pickup unit used in a conventional endoscope is not configured in consideration of a plastic lens's property of being freely formed by molding and being able to be formed in an arbitrary form for the reason that a glass lens has usually been mainly used in a conventional objective lens unit or other reasons.

The present invention has been made in consideration of the above-described circumstances, and has an object to provide an image pickup unit with an objective lens unit using, e.g., a plastic lens which can contribute to an improvement in assemblability and has easily achieved a reduction in optical variations by being configured in consideration of member properties and an endoscope using the image pickup unit.

### Disclosure of Invention

### Means for Solving the Problem

In order to achieve the above-described object, an image pickup unit according to the present invention includes an objective lens unit composed of a plurality of optical members arranged in series with each other in a direction parallel to a single optical axis such that optical axes of the plurality of optical members coincide with each other and a series-connecting portion which unitizes the plurality of optical members and a photoelectric conversion image pickup device which receives an optical image formed by the objective lens unit and performs photoelectric conversion processing, and the series-connecting portion includes a holding portion which holds one of the plurality of optical members, an extending portion which is provided to extend from one end of the holding portion in a direction parallel to the optical axis of the optical member held by the holding portion and is formed to be elastically deformable in a direction almost orthogonal to the optical axis, and an engaging portion which is provided at the extending portion and engages with, of the plurality of optical members, one different from the optical member held by the holding portion.

According to the present invention, it is possible to provide an image pickup unit with an objective lens unit using, e.g., a plastic lens which can contribute to an improvement in assemblability and has easily achieved a reduction in optical variations by being configured in consideration of member properties and an endoscope using the image pickup unit.

### Brief Description of the Drawings

Fig. 1 is a perspective view schematically showing a configuration of an endoscope using an image pickup unit according to a first embodiment of the present invention;
Fig. 2 is a main part enlarged sectional view showing, in an enlarged scale, a part of a section of a distal end portion in the endoscope in Fig. 1;
Fig. 3 is an enlarged sectional view showing, in an enlarged scale, the image pickup unit according to the first embodiment of the present invention;
Fig. 4 is a sectional view taken along line [IV]-[IV] in Fig. 3;
Fig. 5 is a sectional view taken along line [V]-[V] in Fig. 3;
Fig. 6 is a perspective view showing only a cylindrical series-connecting member (series-connecting portion) extracted from the image pickup unit in Fig. 3;
Fig. 7 is a perspective view showing only a first optical member extracted from the image pickup unit in Fig. 3;
Fig. 8 is an enlarged sectional view showing, in an enlarged scale, an image pickup unit according to a second embodiment of the present invention;
Fig. 9 is a sectional view taken along line [IX]-[IX] in Fig. 8;
Fig. 10 is a sectional view taken along line [X]-[X] in Fig. 8;
Fig. 11 is an enlarged sectional view showing, in an enlarged scale, an image pickup unit according to a third embodiment of the present invention;
Fig. 12 is an enlarged sectional view showing, in an enlarged scale, an image pickup unit according to a fourth embodiment of the present invention;
Fig. 13 is an enlarged sectional view showing, in an enlarged scale, an image pickup unit according to a fifth embodiment of the present invention; and
Fig. 14 is an enlarged sectional view showing, in an enlarged scale, an image pickup unit according to a sixth embodiment of the present invention.

### Best Mode for Carrying Out the Invention

The present invention will be described below with reference to illustrated embodiments.

An image pickup unit according to the present invention is used in, e.g., an electronic endoscope (hereinafter simply referred to as an endoscope). Before the embodiments regarding an image pickup unit according to the present invention are described, a schematic configuration of an endoscope in which an image pickup unit according to the present invention is used will first be described below.

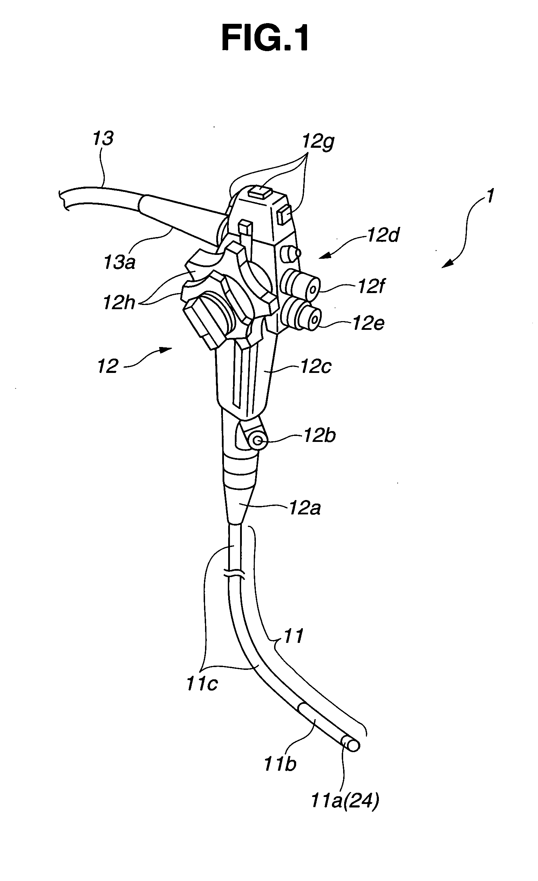

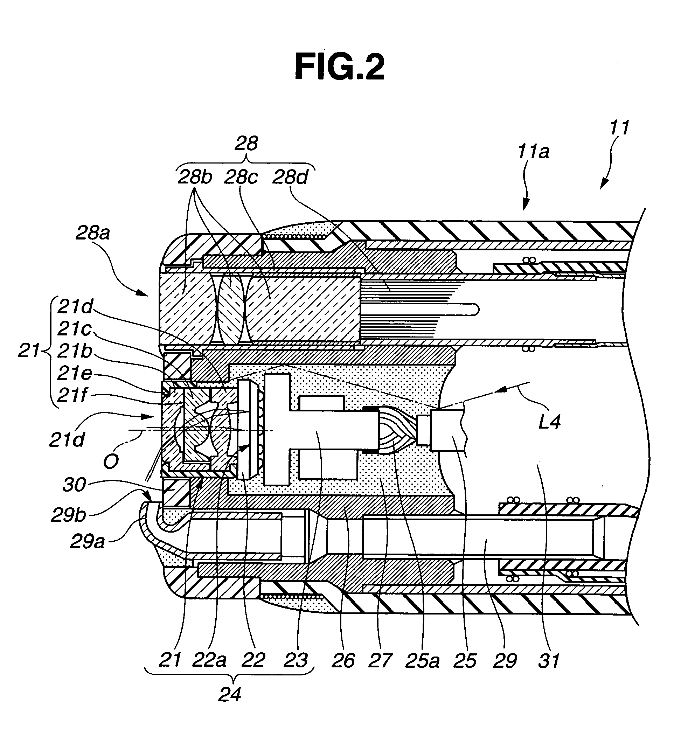

Fig. 1 is a perspective view schematically showing a configuration of an endoscope using an image pickup unit according to a first embodiment of the present invention. Fig. 2 is a main part enlarged sectional view showing, in an enlarged scale, a part of a section of a distal end portion in the endoscope in Fig. 1.

An endoscope 1 to which the present invention is applied is an electronic endoscope including an image pickup unit 24 (see Fig. 2 for details) inside a distal end portion 11a of an insertion portion 11.

The endoscope 1 is mainly composed of the insertion portion 11 in an elongated form to be inserted into a body cavity or the like, an operation portion 12 connected in series with a proximal end side of the insertion portion 11 to be grasped by a user for remote operation, and a universal cable 13 extending from a side portion of the operation portion 12.

In the insertion portion 11, the distal end portion 11a (whose detailed inner configuration will be described later; see Fig. 2), a bending portion 11b, and a flexible tube portion 11c are connected in series with each other in this order from a distal end side. A proximal end side of the flexible tube portion 11c and a distal end side of the operation portion 12 are connected in series with each other via a bend preventing member 12a.

In the operation portion 12, the bend preventing member 12a, a grasping portion 12c, and a main body portion 12d are formed in this order from the distal end side.

A treatment instrument insertion port 12b is provided in the grasping portion 12c of the operation portion 12. The treatment instrument insertion port 12b is connected in series with a treatment instrument insertion channel (not shown) which is inserted through the insertion portion 11 between the proximal end side and the distal end portion 11a. If necessary, a treatment instrument (not shown) or the like is inserted from the treatment instrument insertion port 12b through the treatment instrument insertion channel and is caused to project from a front opening of the distal end portion 11a. This allows various types of treatment and the like.

The main body portion 12d of the operation portion 12 has various operation members including an air-supply and water-supply button 12e, a suction operation button 12f, bending operation knobs 12h, and a plurality of operation members 12g provided on an exterior surface. The operation members are each configured to perform a corresponding function via a mechanism portion, an electrical circuit, and the like (not specifically shown) arranged inside the main body portion 12d.

More specifically, for example, when the air-supply and water-supply button 12e is operated, an instruction signal is generated in response. Upon receipt of the instruction signal, a control circuit (not shown) provided, e.g., inside the main body portion 12d controls a predetermined air supply operation or water supply operation or the like. For example, if water supply control is performed at the time, a water supply pump (not shown) is driven, and fluid in a water supply tank is fed to the distal end portion 11a via a water supply pipe line 29 (see Fig. 2) which is inserted through the insertion portion 11 and is discharged out of an opening 29b (see Fig. 2) of a water supply nozzle 29a provided on a distal end side of the distal end portion 11a.

Operation of the bending operation knob 12h causes a bending mechanism portion (not shown) which is provided inside the main body portion 12d and is composed of a pull wire for a bending operation and the like to act. This allows a bending operation of the bending portion 11b.

A proximal end side of the universal cable 13 is connected in series with the operation portion 12 via a bend preventing portion 13a arranged at the side portion of the operation portion 12, and a distal end side is connected to, e.g., a video processor and a light source apparatus (both not shown) via a connector.

For example, various signal lines including a signal line 25 for image pickup extending from an image pickup device 22 of the image pickup unit 24, light guide fibers 28d, and the like are inserted through the universal cable 13, as will be described later (see Fig. 2). The signal line 25 for image pickup, light guide fibers 28d, and the like pass through the universal cable 13 and operation portion 12 from the video processor and light source apparatus and are inserted through the insertion portion 11 to the distal end portion 11a.

A schematic inner configuration of the distal end portion 11a of the insertion portion 11 in the endoscope 1 described above will be described below.

As shown in Fig. 2, the image pickup unit 24, an illumination optical system 28, the water supply nozzle 29a, the water supply pipe 29, and the like are arranged inside the distal end portion 11a.

The illumination optical system 28 is provided, inside the distal end portion 11a, at a part opposed to an illumination window 28a provided at a front of the distal end portion 11a. The illumination optical system 28 is composed of a plurality of illumination optical members 28b, a holding frame member 28c in a cylindrical form which holds the plurality of illumination optical members 28b, the light guide fibers 28d provided behind the illumination optical members 28b, and the like. With the configuration, an illumination luminous flux from an optical instrument (not shown) reaches the illumination optical system 28 via the light guide fibers 28d and then are applied forward from the illumination window 28a.

The water supply nozzle 29a is arranged to slightly project forward from the distal end portion 11a, and the water supply pipe 29 inserted through the insertion portion 11 is connected in series with a proximal end side of the water supply nozzle 29a. The water supply nozzle 29a is configured to discharge fluid fed via the water supply pipe line 29 toward a front surface of a window 21a for observation which is provided at the front of the distal end portion 11a. For the reason, the opening 29b provided at a distal end of the water supply nozzle 29a is formed toward the front surface of the window 21 a for observation. The image pickup unit 24 is provided, inside the distal end portion 11a, at a side opposed to the window 21a for observation provided at the front of the distal end portion 11a and is mainly composed of an objective lens unit 21, the image pickup device 22, which receives an optical image formed by the objective lens unit 21 at a light-receiving surface 22a and performs photoelectric conversion on the optical image, and a circuit board 23 which is connected to the image pickup device 22 and includes a circuit for performing various types of signal processing. A plurality of signal cables 25a are electrically connected to the circuit board 23, and the signal cable 25 formed by bundling together the plurality of signal cables 25a is inserted through the insertion portion 11, passes through the operation portion 12, and is inserted through the universal cable 13.

The objective lens unit 21 is composed of a plurality of optical members (i.e., a first optical member 21 b, a second optical member 21 c, and a third optical member 21d), a cylindrical member 21e serving as a series-connecting portion for unitizing the plurality of optical members (21b, 21c, and 21d) by connecting the plurality of optical members in series with each other in a predetermined arrangement, a diaphragm member 21f which regulates the amount of incident light and sets a depth of field, and the like.

Note that the plurality of optical members 21b, 21c, and 21d are arranged such that optical axes of the plurality of optical members almost coincide with each other. The arrangement defines an optical axis O of the objective lens unit 21. In other words, the plurality of optical members 21b, 21c, and 21d are arranged along the optical axis O defined by causing the optical axes to almost coincide with each other.

The window 21a for observation is arranged at a distal end portion of the distal end portion 11a and is formed from a distal end cover member 30 made of, e.g., plastic. A distal end portion of the image pickup unit 24 fits in the distal end cover member 30. A distal end side of the image pickup unit 24 is fixedly supported by the distal end cover member 30.

A distal end holding member 26 is connected in series with a rear side (a surface close to a proximal end) of the distal end cover member 30. A main portion of the image pickup unit 24 is housed and arranged in a space inside the distal end holding member 26. In the state, the distal end holding member 26 is filled with an adhesive agent 27. The adhesive agent 27 fixes the image pickup unit 24 inside the distal end holding member 26.

A tubular space 31 extending through the insertion portion 11 is formed behind (on a proximal end side of) the distal end holding member 26, and the signal cable 25 and the like are inserted through the space 31.

A detailed configuration of the image pickup unit 24 according to the present embodiment used in the endoscope 1 with the above-described configuration will be described below.

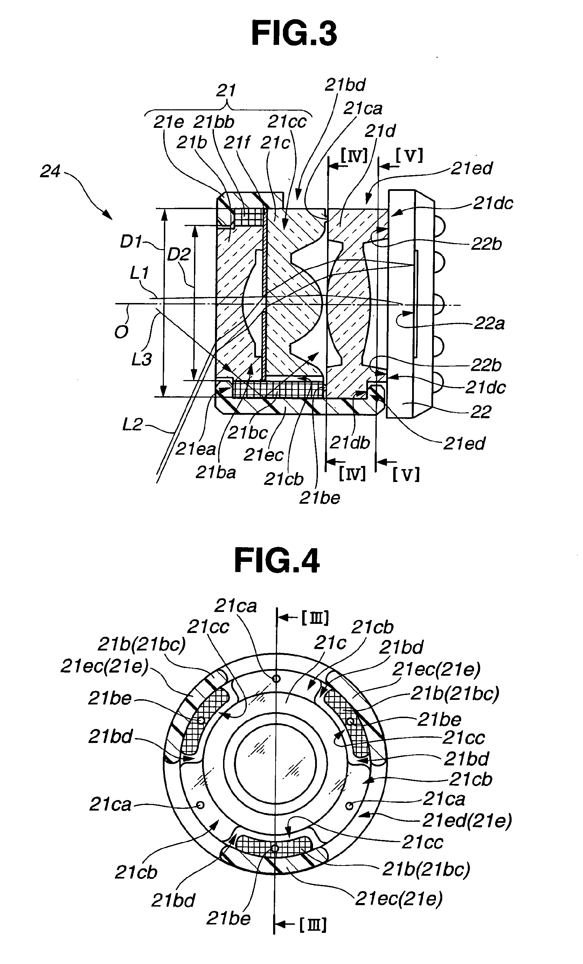

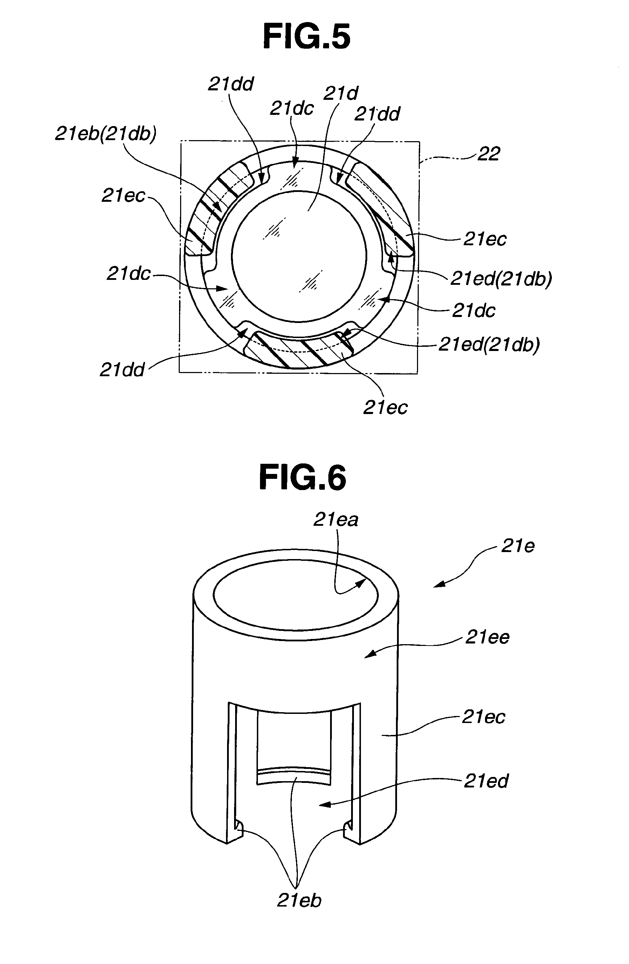

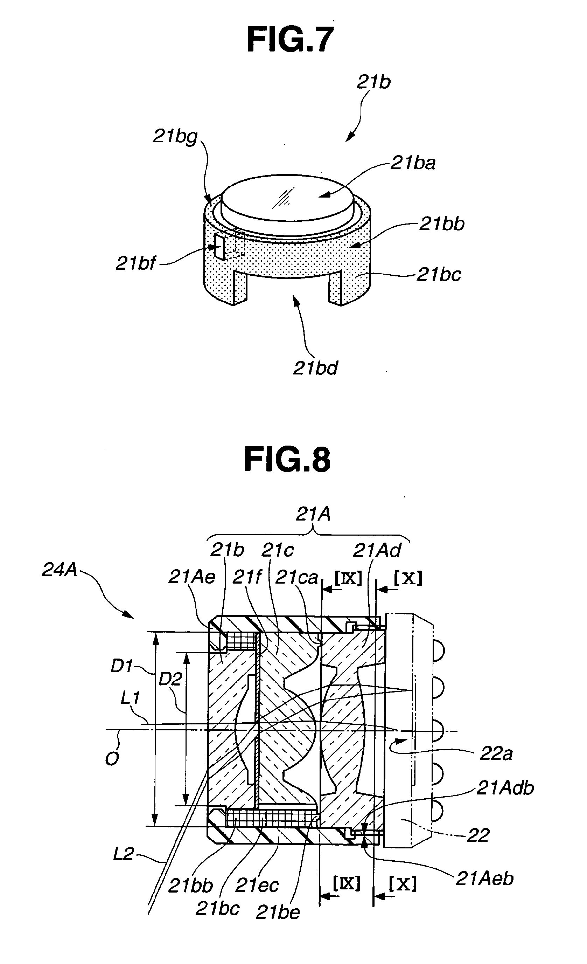

Figs. 3 to 7 are views showing the image pickup unit according to the first embodiment of the present invention. Of the figures, Fig. 3 is an enlarged sectional view showing, in an enlarged scale, the image pickup unit according to the present embodiment. Note that Fig. 3 shows a section taken along line [III]-[III] in Fig. 4. Fig. 4 is a sectional view taken along line [IV]-[IV] in Fig. 3. Fig. 5 is a sectional view taken along line [V]-[V] in Fig. 3. Fig. 6 is a perspective view showing only the cylindrical series-connecting member (series-connecting portion) extracted from the image pickup unit in Fig. 3. Fig. 7 is a perspective view showing only the first optical member extracted from the image pickup unit in Fig. 3.

As described above, the image pickup unit 24 according to the present embodiment is mainly composed of an objective lens unit 21 made up of the plurality of optical members (the first optical member 21 b, the second optical member 21 c, and the third optical member 21d), the cylindrical member 21e serving as the series-connecting portion for unitizing the plurality of optical members (21 b, 21 c, and 21d) by connecting the plurality of optical members in series with each other in the predetermined arrangement, the diaphragm member 21 f having a function of performing regulation of the amount of incident light and other processes, and the like and the image pickup device 22, which is arranged behind the objective lens unit 21, has the light-receiving surface 22a for receiving an optical image formed by the objective lens unit 21, and serves as a photoelectric conversion image pickup device for performing photoelectric conversion processing based on an optical image received at the light-receiving surface 22a and generating an image signal.

Note that, as described above, the circuit board 23, on which the electrical circuit for receiving an output signal from the image pickup device 22 and performing various types of signal processing and the like are mounted, is electrically connected to the image pickup device 22 (see Fig. 2).

The plurality of optical members, i.e., the first optical member 21b, the second optical member 21 c, and the third optical member 21 d are each formed of, e.g., a plastic-molded member. As described above, the plurality of optical members are arranged in series with each other along the optical axis O such that the optical axes almost coincide with each other.

As shown in Fig. 6, the cylindrical member 21e is composed of a holding portion 21ee for holding one of the plurality of optical members, i.e., the first optical member 21 b and extending portions 21ec which are provided to extend from one end (a rear end side) of the holding portion 21ee in a direction parallel to the optical axis O of the first optical member 21b held by the holding portion 2lee. Note that the cylindrical member 21e is also formed of, e.g., a plastic-molded member, like the plurality of optical members.

The extending portions 21ec are arm-like parts provided at three positions almost equally spaced in a circumferential direction in a section of the cylindrical member 21e (see Fig. 4). As for the three extending portions 21ec, a notch portion 21ed which is a notch-like space is formed between adjacent ones of the extending portions 21 ec (see Figs. 6 and 4). The extending portions 21 ec are each formed to be elastically deformable in a direction almost orthogonal to the optical axis O.

As shown in Figs. 6 and 3, an engaging portion 21eb in a hooked shape engages with a predetermined part near a proximal end (an engaged portion 21db shown in Fig. 3) of the third optical member 21d of the plurality of optical members is formed at a distal end portion of each extending portion 21 ec.

In the present embodiment, of the plurality of optical members constituting the objective lens unit 21 in the image pickup unit 24, one held by the holding portion 21ee is the first optical member 21b. Of the plurality of optical members constituting the objective lens unit 21, one different from the optical member held by the holding portion 21ee (the first optical member 21b) is the third optical member 21 d. Accordingly, a different one with which the engaging portion 21eb of each extending portion 21 ec engages is the third optical member 21 d.

The holding portion 21ee of the cylindrical member 21e is arranged to cover an outer edge of the first optical member 21 b of the plurality of optical members. For the reason, a step 21ea is formed on a distal end side of the cylindrical member 21e, as shown in Fig. 3 (and Fig. 6). With the configuration, a front opening size D2 of the cylindrical member 21e is set to be smaller than an inner diameter D1 of the cylindrical member 21e (D 1> D2), as shown in Fig. 3.

As shown in Fig. 4, a stepped portion 21bg is also formed on a distal end side of the first optical member 21 b corresponding to the stepped portion 21 ea. With the configuration, when the first optical member 21b is inserted into the holding portion 21ee of the cylindrical member 21e, and the holding portion 21ee enters a state of holding the first optical member 21 b to cover the outer edge, the stepped portion 21 bg of the first optical member 21 b and the step 21ea of the cylindrical member 21 e abut on each other. At the time, the first optical member 21b is positioned at a predetermined position of an inner portion of the cylindrical member 21 e.

The first optical member 21b is formed to have, in a section of the first optical member 21b (see Fig. 4), arm portions 21bc (see also Fig. 7) at three positions almost equally spaced in the circumferential direction, like the cylindrical member 21e. A notch portion 21bd is formed in a space between adjacent ones of the arm portions 21bc (see Fig. 7). When the first optical member 21b is incorporated into the cylindrical member 21e, the arm portions 21bc are arranged inside the extending portions 21ec, as shown in Fig. 4.

Note that when the image pickup unit 24 is in an assembled state, a distal end surface of each arm portion 21bc of the first optical member 21 b is configured to abut on a predetermined part of a front surface of the third optical member 21d, as will be described later. For the reason, a projection-like vertical effect generating portion 21be which positions the third optical member 21d relative to the first optical member 21b is formed at the distal end surface of each arm portion 21bc.

As shown in Fig. 7, the first optical member 21 b is composed of an optical portion 21ba made of a transparent resin and a main body portion 21 bb made of a black resin (indicated by a polka dot pattern in Fig. 7; note that the same is indicated by a grid pattern in Figs. 3 and 4). The main body portion 21bb is composed of a part formed to cover an outer edge of the optical portion 21ba and the three arm portions 21bc extending from the part. The optical portion 21ba and main body portion 21bb are integrally formed with each other.

At the time of molding of the first optical member 21 b, for example, a method is used of molding the main body portion 21bb from black resin and then molding the optical portion 21ba by injecting a transparent resin from a hole 21bf (see Fig. 7) drilled in a side portion of the main body portion 21 bb into the main body portion 21bb.

The above-described formation of a part of the first optical member 21b using the black resin or the like makes it possible to block a stray light beam which cannot be blocked by the diaphragm member 21f (e.g., a stray light beam indicated by an arrow L3 in Fig. 3).

Note that although an unnecessary light beam may come incident via the hole 21bf in the case, incident light from the hole 21bf can be reliably blocked by making some contrivance such that the holding portion 21ee of the cylindrical member 21e or the like can close the hole 21bf when the first optical member 21 b is incorporated into the cylindrical member 21e.

The diaphragm member 21f provided to regulate an opening size of the first optical member 21 b and regulate the amount of incident light is arranged on a rear side of the first optical member 21 b.

The second optical member 21 c is arranged behind the first optical member 21 b such that the diaphragm member 21f is sandwiched between the first optical member 21b and the second optical member 21 c.

As shown in Fig. 4, the second optical member 21c is formed to have three convex portions 21cb projecting outward at positions almost equally spaced in the circumferential direction in a section of the second optical member 21 c. A notch portion 21cc is formed in a space between adjacent ones of the convex portions 21 cb.

When the second optical member 21c is incorporated into the first optical member 21 b, each arm portion 21bc of the first optical member 21 b is arranged in the corresponding notch portion 21cc of the second optical member 21 c, and each convex portion 21cb of the second optical member 21b is arranged in the corresponding notch portion 21bd of the first optical member 2 1 b, as shown in Fig. 4.

A projection-like vertical effect generating portion 21ca projecting backward is provided at a rear end surface of each convex portion 21 cb of the second optical member 21c (see Figs. 3 and 4). Each vertical effect generating portion 21ca is configured to abut on a predetermined portion near an outer edge of the front surface of the third optical member 21d when the third optical member 21d is incorporated into the second optical member 21 c.

That is, when the third optical member 21d is incorporated into the second optical member 21 c, a rear surface of the second optical member 21 c and the front surface of the third optical member 21d abut on each other. Provision of the vertical effect generating portions 21ca brings both the optical members 21 c and 21 d into point contact with each other at the time. With the configuration, the third optical member 21 d is positioned relative to the second optical member 21 c.

Note that although the present embodiment is configured such that the vertical effect generating portions 21ca are provided at the second optical member 21 c, the present invention is not limited to this. The present embodiment may be configured such that a vertical effect generating portion is provided at the third optical member 21 d. In the case as well, exactly same effects can be achieved.

The third optical member 21d is formed to have an almost the same shape as the second optical member 21c. That is, the third optical member 21d is formed to have three convex portions 21dc projecting outward at positions almost equally spaced in the circumferential direction in a section of the third optical member 21d, and a notch portion 21dd is formed in a space between adjacent ones of the projecting portions 21 dc, as shown in Fig. 5.

When the third optical member 21d is incorporated into the second optical member 21c, each extending portion 21ec of the cylindrical member 21 e is arranged in the corresponding notch portion 21 dd of the third optical member 21 d, as shown in Fig. 5. Each extending portion 21ec of the cylindrical member 21e is arranged in the corresponding notch portion 21bd of the third optical member 21d.

A rear end surface of the third optical member 21 d abuts on a part near an outer edge of a front surface of the image pickup device 22 provided behind the third optical member 21d. The third optical member 21 d and image pickup device 22 are adhesively fixed at the position using, e.g., an adhesive agent or the like. Note that a position of the objective lens unit 21 relative to the image pickup device 22 is defined at the time such that the optical axis O of the objective lens unit 21 almost coincide with an almost central point of the light-receiving surface 22a of the image pickup device 22.

The engaging portion 21 eb of each extending portion 21 ec is configured to be locked by the corresponding engaged portion 21db of the third optical member 21d. For the reason, an inner diameter of each engaging portion 2 1 eb of the cylindrical member 21e is set to be smaller than an outer diameter of an outermost perimeter of the third optical member 21d, and each extending portion 2 1 ec of the cylindrical member 21 e where the corresponding engaging portion 21 eb is provided is formed to be elastically deformable in a direction almost orthogonal to an extending direction, i.e., a direction almost parallel to the optical axis O.

With the configuration, the third optical member 21d can pass through an interior of the engaging portions 2 1 eb of the cylindrical member 21e while elastically deforming the engaging portions 21 eb and can be arranged at a predetermined position.

The cylindrical member 21e is configured to also hold the third optical member 21d by the engaging portions 21 eb engaging with the engaged portions 21 db of the third optical member 21d while the first optical member 21 b is held, and the second optical member 21 c is sandwiched.

The objective lens unit 21 in the image pickup unit 24 with the above-described configuration is assembled in a manner below.

First, the first optical member 21 b is inserted from a proximal end side of the cylindrical member 21e. At the time, the first optical member 21b is arranged such that each arm portion 21bc of the first optical member 21 b is located inside the corresponding extending portion 21 ec of the cylindrical member 21e. The stepped portion 21 bg of the first optical member 21 b is placed in a state of abutting on the step 21ea of the cylindrical member 21 e. With the operations, the optical portion 21ba of the first optical member 21b fits into the distal end side of the cylindrical member 21 e to be exposed, and the main body portion 21 bb of the first optical member 21b is held by the holding portion 21ee of the cylindrical member 21e such that the holding portion 21ee covers an outer edge. When the first optical member 21b enters the state, the first optical member 21b is positioned at a predetermined position of the cylindrical member 21. Note that the optical axis of the first optical member 21 b is set to be almost parallel to the extending portions 21ec of the cylindrical member 21e.

The second optical member 21 c is inserted from the proximal end side into the cylindrical member 21e in the state. At the time, each convex portion 21 cb of the second optical member 21 c is set to be arranged in the corresponding notch portion 21bd of the first optical member 21 b, and each arm portion 21bc of the first optical member 21 b is set to be arranged in the corresponding notch portion 21cc of the second optical member 21c, as shown in Fig. 4. A front surface of the second optical member 21c is set to abut on a rear side of the diaphragm member 21f arranged on the rear side of the first optical member 21 b, as shown in Fig. 3. At the time, the front surface of the second optical member 21c and a rear surface of the first optical member 21b have abutment surfaces almost parallel to each other, and this causes the optical axes of both the optical members to almost coincide with each other.

The third optical member 21d is inserted from the proximal end side into the cylindrical member 21e in the state. At the time, the third optical member 21 d is arranged such that each convex portion 21 dc of the third optical member 21d overlaps the corresponding convex portion 21 cb of the second optical member 21 c, as shown in Fig. 5. Simultaneously, each extending portion 21ec of the cylindrical member 21e is set to be arranged in the corresponding notch portion 21 dd of the third optical member 21d. Each extending portion 21ec of the cylindrical member 21e is arranged in the corresponding notch portion 21bd of the third optical member 21d. In the case, each engaging portion 21eb of the cylindrical member 21e is caused to engage with the corresponding engaged portion 21 db of the third optical member 21d.

Each extending portion 21ec of the cylindrical member 21e is configured to be elastically deformable in a direction almost orthogonal to the optical axis O. Accordingly, when the third optical member 21d travels in a direction parallel to the optical axis O, each extending portion 21ec is pressed by the outer edge of the third optical member 21d in a direction almost orthogonal to the optical axis O, away from the optical axis O. This allows the third optical member 21d to pass through the interior of the engaging portions 21 eb. When the engaged portions 21 db and engaging portions 21eb engage with each other, each extending portion 21ec is restored to a predetermined normal state, i.e., a state almost parallel to the optical axis O by the extending portion 21 ec's own elastic restoring force.

At the time, each predetermined part near the outer edge of the front surface of the third optical member 21d abuts on the corresponding vertical effect generating portion 21be formed at the distal end surface of the arm portion 21bc of the first optical member 21b. This positions the third optical member 21d relative to the first optical member 21 b. Simultaneously, each predetermined part near the outer edge of the front surface of the third optical member 21d abuts on the corresponding vertical effect generating portion 21ca formed at the rear end surface of the convex portion 21 cb of the second optical member 21 c. This positions the third optical member 21d relative to the second optical member 21b.

In the above-described manner, the three optical members 21b, 21c, and 21d are housed in series with each other within the cylindrical member 21e, and the objective lens unit 21, into which the cylindrical member 21e and the plurality of optical members (21 b, 21 c, and 21d) are unitized, is formed.

The optical axis O of the objective lens unit 21 formed by the unitization almost coincides with the optical axes of the optical members 21 b, 21 c, and 21 d.

The objective lens unit 21 is fixedly arranged on a front side of the image pickup device 22. In the case, a part near an outer edge of the rear end surface of the third optical member 21d and the predetermined part near the outer edge of a front surface 22b of the image pickup device 22 are adhesively fixed by, e.g., an ultraviolet curable adhesive agent.

At the time, relative positioning between the objective lens unit 21 and the image pickup device 22 is performed such that the optical axis O of the objective lens unit 21 almost coincides with the central point of the light-receiving surface 22a of the image pickup device 22 and such that horizontal and vertical directions of an image formed by the objective lens unit 21 and horizontal and vertical directions of an almost rectangularly shaped image pickup region defined by the light-receiving surface 22a almost coincide with each other.

For the reason, an optical image to be formed by the objective lens unit 21 is formed on the light-receiving surface 22a of the image pickup device 22 provided behind the third optical member 21d. At the time, light incident on the objective lens unit 21 passes through the diaphragm member 21 f and focuses on the image pickup device 22. Reference characters L1 and L2 in Fig. 3 denote typical light beams having passed through the diaphragm member 21 f.

In response, the image pickup device 22 performs predetermined photoelectric conversion processing based on the optical image received by the light-receiving surface 22a and generates an image signal. The image signal generated by the image pickup device 22 is outputted to, e.g., the circuit board 23. After the image signal is subjected to various types of signal processing by the electric circuit on the circuit board 23, the image signal is outputted toward the operation portion 12 via the signal cable 25. Finally, the image signal is transmitted to the video processor and the like (not shown) via the universal cable 13.

Note that the light beam indicated by reference characters L3 in Fig. 3 enters the objective lens unit 21, in addition to the light beams L1 and L2 focused on the light-receiving surface 22a of the image pickup device 22. The light beam L3 is a noise component called a stray light beam. If a light beam serving as a noise component reaches the light-receiving surface 22a of the image pickup device 22, the light beam causes degradation in image quality.

For example, if the light beam L3 is reflected by an outer wall and the arm portions 21bc of the first optical member 21 b and enters the second optical member 2 1 c and the like, as shown in Fig. 3, the light beam L3 may reach the light-receiving surface 22a.

Accordingly, the image pickup unit 24 according to the present embodiment blocks the stray light beam L3 by providing the diaphragm member 21 f on the rear side of the first optical member 21 b and forming a part (see a part shaded with the grid pattern in Fig. 3) of each arm portion 21bc of the first optical member 2 1 b using the black resin or the like.

As shown in Fig. 1, in the distal end portion 11 a of the endoscope insertion portion 11, leakage light L4 from the light guide fibers 28d or the like may enter the objective lens unit 21 from a back side of the image pickup unit 24.

In consideration of the possibility, according to the present embodiment, a part of the distal end holding member 26, which fixedly supports the image pickup unit 24, is configured to cover the notch portions 21ed of the cylindrical member 21e in the objective lens unit 21. Additionally, a black adhesive agent is used as the adhesive agent 27, with which the distal end holding member 26 is to be filled. This inhibits the leakage light L4 from entering the image pickup unit 24.

Provisions for reliable light blocking have been made for the image pickup unit 24 according to the present embodiment such that no unnecessary light beam enters the image pickup unit 24.

As has been described above, according to the first embodiment, the plurality of optical members 21 b, 2 1 c, and 21d and the cylindrical member 21e are formed using a molded member, and a shape of the cylindrical member 21e is inventively designed such that positioning of the plurality of optical members 21b, 21 c, and 21 d in a direction parallel to the optical axis O using the cylindrical member 21 e can be performed simultaneously with fixed holding of the plurality of optical members 21b, 21c, and 21d by the same cylindrical member 21e. Accordingly, one member (the cylindrical member 21 e) can be configured to be responsible for fixed holding and positioning of optical members. The present embodiment can thus contribute to a configuration simplification and to an improvement in assemblability.

Positioning of the first optical member 21 b relative to the third optical member 21 d is performed by the vertical effect generating portions 21 e while positioning of the second optical member 21 c relative to the third optical member 21d is performed by the vertical effect generating portions 21 ca. That is, positioning of each of the plurality of optical members 21 b, 21 c, and 21d in a direction parallel to the optical axis O relative to the third optical member 21d serving as a reference is independently performed. Accordingly, errors are not accumulated at the time of positioning of a plurality of optical members, and higher positioning accuracy can be ensured. It is thus possible to easily achieve a reduction in optical variations and contribute to an improvement in optical performance.

An image pickup unit according to a second embodiment of the present invention will be described below with reference to Figs. 8, 9, and 10.

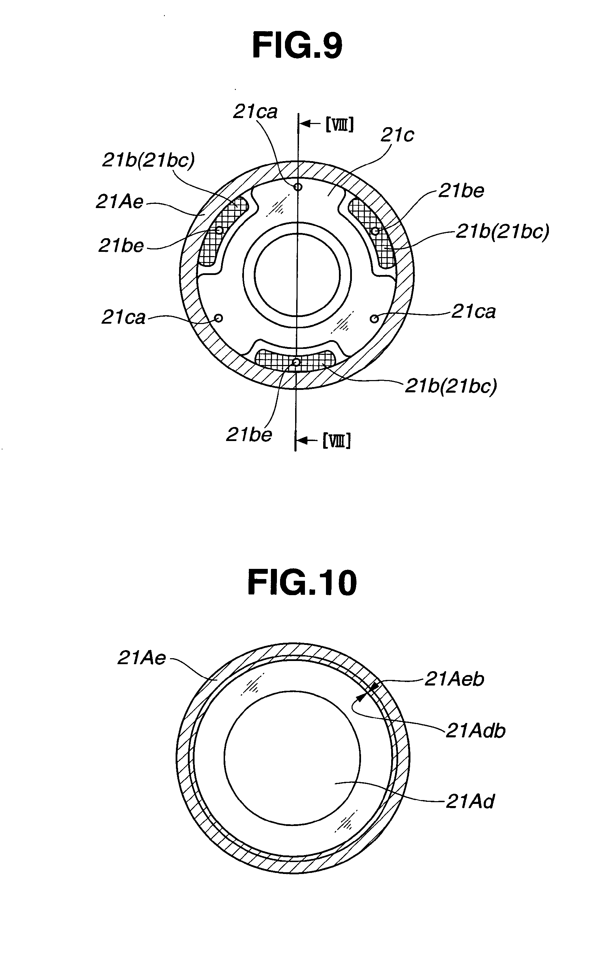

Fig. 8 is an enlarged sectional view showing, in an enlarged scale, the image pickup unit according to the second embodiment of the present invention. Note that Fig. 8 shows a section taken along line [VIII]-[VIII] in Fig. 9. Fig. 9 is a sectional view taken along line [IX]-[IX] in Fig. 8. Fig. 10 is a sectional view taken along line [X]-[X] in Fig. 8.

A basic configuration of an image pickup unit 24A according to the present embodiment is almost the same as the first embodiment described above and is different in a configuration of a part where a third optical member 21Ad and a cylindrical member 21Ae are connected in series with each other. That is, in the first embodiment, the third optical member 21d and cylindrical member 21e are configured to be connected in series with each other by causing the engaging portion 21 eb of each extending portion 21ec of the cylindrical member 21e to engage with the corresponding engaged portion 21db of the third optical member 21d.

On the other hand, in the image pickup unit 24A according to the present embodiment, the third optical member 21 Ad and cylindrical member 21Ae are configured to be connected in series with each other by forming a threaded portion 21 Aeb composed of a male thread or female thread on an inner peripheral surface of a distal end portion of the cylindrical member 21Ae, forming a threaded portion 21Adb composed of a female thread or male thread which fits onto or into the threaded portion 21Aeb at a part near a proximal end of the third optical member 21 Ad on an outer peripheral surface, and causing the threaded portions 21Aeb and 21Adb to fit together.

For the reason, the cylindrical member 21Ae in the image pickup unit 24A according to the present embodiment is formed in whole to be cylindrical, and the extending portions 21ec and notch portions 21ed formed at the cylindrical member 21e according to the first embodiment are not provided. Note that, like the cylindrical member 21 e according to the first embodiment, a shape of a distal end side of the cylindrical member 21 Ae is shaped to suit a shape of a distal end side of a first optical member 21 b such that a front opening size D2 of the cylindrical member 21Ae is smaller than an inner diameter D1 of the cylindrical member 2 1 Ae (D 1> D2).

Other configurations are the same as the first embodiment.

The image pickup unit 24A according to the second embodiment with the above-described configuration is assembled in an almost the same procedure as the image pickup unit 24 according to the first embodiment.

In the case, after the first optical member 21b is first inserted into the cylindrical member 21 Ae from a proximal end side, a second optical member 21 c is inserted into the cylindrical member 21 Ae from the proximal end side, like the first embodiment. The third optical member 21Ad is then inserted into the cylindrical member 21Ae in the state from the proximal end side. At the time, the threaded portion 2 1 Adb at the part near the proximal end of the third optical member 21Ad on the outer peripheral surface is caused to fit onto or into the threaded portion 21Aeb on the inner peripheral surface of the distal end portion of the cylindrical member 21 Ae. With the operation, the threaded portions 21Adb and 21Aeb are connected in series with each other.

Other procedures for assembling the image pickup unit 24A are the same as the first embodiment.

As has been described above, according to the second embodiment, the same effects as the first embodiment can be achieved. Additionally, since thread connection, which causes the threaded portions to fit together, is used to connect the third optical member 21Ad and cylindrical member 21Ae in series with each other, the present embodiment can contribute to a further improvement in assemblability.

According to a configuration of the present embodiment, since the cylindrical member 21 Ae can be formed without providing a notch portion or the like, the cylindrical member 21 Ae can reliably cover outer edges of the plurality of optical members arranged inside. It is thus possible to reliably block entry of a light beam unnecessary for forming an image by an objective lens unit 21A composed of the plurality of optical members.

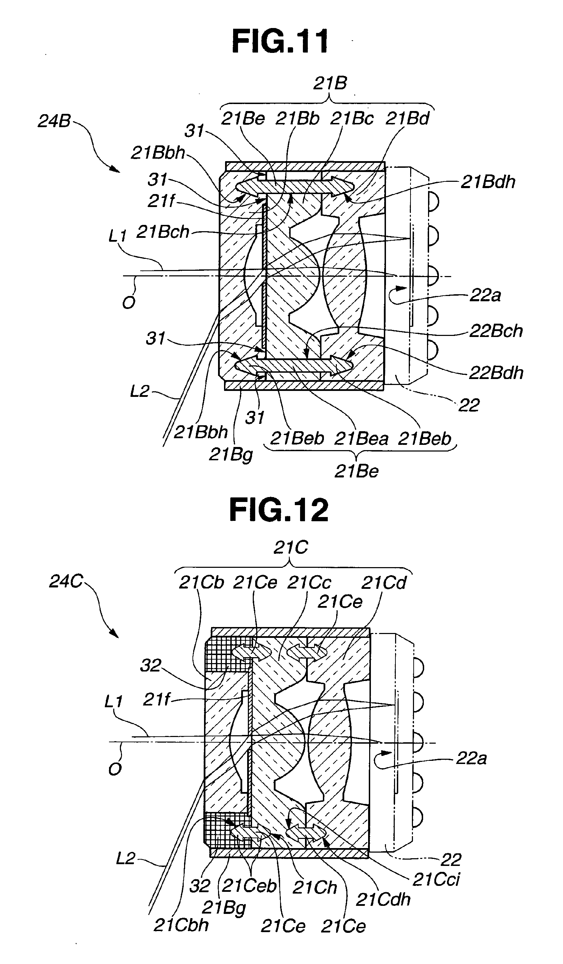

An image pickup unit according to a third embodiment of the present invention will be described below with reference to Fig. 11.

Fig. 11 is an enlarged sectional view showing, in an enlarged scale, the image pickup unit according to the third embodiment of the present invention.

As shown in Fig. 11, a basic configuration of an image pickup unit 24B according to the present embodiment is almost the same as the first and second embodiments described above and is different in a configuration of a series-connecting portion for unitizing a plurality of optical members by connecting the plurality of optical members in series with each other.

That is, in the first and second embodiments, the third optical member (21 d or 21Ad) and the cylindrical member serving as the series-connecting portion (21e or 21Ae) are configured to be connected in series with each other by causing the engaging portions 21eb or the threaded portion 21Adb provided in the cylindrical member (21e or 21Ae) to engage with or fit into or onto the engaged portions 21db or the threaded portion 21Aeb of the third optical member.

On the other hand, the image pickup unit 24B according to the present embodiment is configured using a plurality of series-connecting members 21Be serving as series-connecting portions, each of which is arranged to extend through a plurality of optical members and has one end engaging with a first optical member 21Bb and the other end engaging with a third optical member 21Bd, instead of the cylindrical member (21e or 2 1 Ae) serving as the series-connecting portion in the first and second embodiments.

The plurality of series-connecting members 21Be are each formed to have a main body portion 21Bea formed in an elongated columnar shape and engaging portions 21Beb in an almost conical shape formed at two ends of the main body portion 21Bea. Each series-connecting member 21Be is formed of, e.g., a wholly black resin member. At least each engaging portion 21 Beb is formed to be elastic.

The plurality of series-connecting members 21Be are arranged at, e.g., three positions equally spaced in a circumferential direction near an outer edge of an objective lens unit 21B.

Engaged holes or through holes are formed at corresponding parts of each of a plurality of optical members constituting the objective lens unit 21B to correspond to the series-connecting members 21Be.

More specifically, an engaged hole 21Bbh with which the corresponding engaging portion 21 Beb at one end of the series-connecting member 21Be engages is drilled in the first optical member 21Bb. The engaged hole 21Bbh is formed to have an almost same shape to suit the almost conical shape of the engaging portion 21Beb at the one end of the series-connecting member 21Be.

A through hole 21Bch which extends at a part near an outer edge between a front surface and a rear surface in a direction parallel to an optical axis O and through which the main body portion 21Bea of the corresponding series-connecting member 21Be is inserted is formed in a second optical member 21Bc.

An engaged hole 21Bdh with which the corresponding engaging portion 21Beb at the other end of the series-connecting member 21Be engages is drilled in the third optical member 21Bd. The engaged hole 21 Bdh is formed to have an almost same shape to suit the almost conical shape of the engaging portion 21Beb at the other end of the series-connecting member 21Be.

A diaphragm member 21f is arranged to be sandwiched between a rear side of the first optical member 21Bb and a front side of the second optical member 21Bc, like the embodiments described above. The diaphragm member 21f has, near the optical axis O, a hole with a predetermined size formed to let light beams pass through within a predetermined range and has an outermost edge arranged near a part where each series-connecting member 21Be is arranged.

With the above-described configuration, an incident light beam from a front surface of the first optical member 21Bb may enter the second optical member 21Bc via a part outside the outermost perimeter of the diaphragm member 21 f and become a stray light beam.

Accordingly, in the present embodiment, a part outside the outermost perimeter of the diaphragm member 21f is subjected to, e.g., coloring with a light-shielding black color on the rear side of the first optical member 21Bb, on the front side of the second optical member 21Bc, or on both the sides. This inhibits an unnecessary light beam from entering via the part.

The present embodiment is configured without the cylindrical member (21e or 21Ae) functioning as the series-connecting portion in the first and second embodiments described above. The cylindrical member (21e or 21Ae) also functions as a light-shielding member.

Accordingly, in the present embodiment configured without the cylindrical member, a light-shielding cylindrical member 21Bg in an almost cylindrical shape is arranged to cover a whole exterior of the objective lens unit 21B in order to block entry of an unnecessary light beam from a side into the objective lens unit 21B. The light-shielding cylindrical member 21 Bg not only serves for light blocking but also functions as a fixing frame which fixedly holds an outer peripheral side of the plurality of optical members.

Note that the three optical members 21Bb, 21Bc, and 21Bd are series-connected and fixed by the series-connecting members 21Be at the time of formation of the objective lens unit 21B as a unit. Positioning of each optical member in a front-back direction (a direction parallel to the optical axis O) in the case is performed using abutment surfaces of the adjacent optical members. Accordingly, the vertical effect generating portions of the plurality of optical members in the embodiments are not formed in the plurality of optical members according to the present embodiment.

Other configurations are almost the same as the first and second embodiments described above.

The image pickup unit 24B according to the third embodiment with the above-described configuration is assembled in a procedure below.

Each series-connecting member 21Be is adhesively fixed to the second optical member 21Bc while the main body portion 21Bea of the series-connecting member 21Be is inserted through the corresponding through hole 21Bch of the second optical member 21Bc. The first optical member 21Bb is arranged toward the front side of the second optical member 21Bc.

More specifically, each engaging portion 21Beb at one end of the series-connecting member 21Be is fit into the corresponding engaged hole 21 Bbh of the first optical member 21Bb. At the time, the engaging portion 21Beb of the series-connecting member 21Be is press-fit into the engaged hole 21 Bbh while the engaging portion 21Beb is compressed against elastic force of the engaging portion 21 Beb. When the engaging portion 21 Beb is caused to engage with the engaged hole Bbh, the engaging portion 21 Beb is restored to an original shape by the engaging portion 21Beb's own elastic force. This places the engaging portion 21 Beb into a state of not falling out easily from the engaged hole 21Bbh. In the state, the first optical member 21Bb and second optical member 21Bc are subjected to, e.g., adhesive fixation.

Series connection and fixation of the second optical member 21Bc and third optical member 21Bd is performed in the exactly the same procedure as described above.

The objective lens unit 21B, into which the plurality of optical members 21Bb, 21Be, and 21Bd are unitized by series connection and fixation, is formed in the above-described manner. The light-shielding cylindrical member 21 Bg is arranged for the objective lens unit 21B to cover the whole exterior of the objective lens unit 21B.

The objective lens unit 21B is adhesively fixed to a predetermined position on a front side of an image pickup device 22. With the operation, the image pickup unit 24B according to the present embodiment is assembled.

As has been described above, according to the third embodiment, almost the same effects as the first and second embodiments can be achieved. Additionally, it is possible to contribute to a further improvement in assemblability by reliably performing positioning and fixation of each optical member while a shape of the series-connecting members 21Be, which series-connects and fixes the plurality of optical members, is further simplified.

An image pickup unit according to a fourth embodiment of the present invention will be described below with reference to Fig. 12.

Fig. 12 is an enlarged sectional view showing, in an enlarged scale, the image pickup unit according to the fourth embodiment of the present invention.

As shown in Fig. 12, a basic configuration of an image pickup unit 24C according to the present embodiment is almost the same as the third embodiment described above and is slightly different in a configuration of a series-connecting portion for unitizing a plurality of optical members by connecting the plurality of optical members in series with each other.

That is, in the third embodiment, the plurality of optical members 21Bb, 21Bc, and 21Bd are configured to be simultaneously connectable in series with each other using one series-connecting member 21Be.

On the other hand, in the image pickup unit 24C according to the present embodiment, three optical members 21 Cb, 21 Cc, and 21 Cd are unitized by individually connecting each pair of ones adjacent in a direction parallel to an optical axis O of the optical members using a plurality of series-connecting members 2 1 Ce.

The plurality of series-connecting members 21 Ce according to the present embodiment are almost the same in basic shape as the series-connecting members 21Be according to the third embodiment and are formed in an elongated columnar shape to have engaging portions 21 Ceb in an almost conical shape formed at two ends. However, each series-connecting member 21 Ce according to the present embodiment is formed to be only long enough to connect adjacent ones of the optical members in series with each other. That is, a total length of each pair of series-connecting members 21 Ce according to the present embodiment is shorter than a length of each series-connecting member 21Be according to the third embodiment by a length of an optical member absent in the optical member 21 Cc which extends through and holds an optical member between optical members engaging with and holding the optical member at two ends.

For example, three series-connecting members 21Ce are used to series-connect and fix the first optical member 21 Cb and second optical member 21 Cc, and the series-connecting members 21 Ce are arranged at three positions equally spaced in a circumferential direction.

Similarly, three series-connecting members 21 Ce are used to series-connect and fix the second optical member 21Cc and third optical member 21Cd, and the series-connecting members 21 Ce are arranged at three positions equally spaced in the circumferential direction.

Note that each series-connecting member 21 Ce is the same as the series-connecting member 21Be according to the third embodiment in that the series-connecting member 21 Ce is formed of, e.g., a wholly black resin member, and at least each engaging portion 21 Ceb is formed to be elastic.

Engaged holes with a shape suited to the engaging portions 21 Ceb of the series-connecting members 21 Ce are formed at corresponding parts of each of the three optical members 21 Cb, 21 Cc, and 21Cd.

More specifically, an engaged hole 21Cbh with which the corresponding engaging portion 21Ceb at one end of the series-connecting member 2 1 Ce for series connection with the second optical member 21Cc engages is drilled in the first optical member 21 Cb. The engaged hole 21 Cbh is formed to have an almost same shape to suit the almost conical shape of the engaging portion 21 Ceb at the one end of the series-connecting member 21Ce.

A front engaged hole 21 Cch with which the corresponding engaging portion 21 Ceb at the other end of the series-connecting member 21 Ce for series connection with the first optical member 21 Cb engages is drilled in the second optical member 21Cc. The front engaged hole 21 Cch is formed to have an almost same shape to suit the almost conical shape of the engaging portion 21 Ceb at the other end of the series-connecting member 21 Ce.

A rear engaged hole 21 Cci with which the corresponding engaging portion 21 Ceb at one end of the series-connecting member 21 Ce for series connection with the third optical member 21 Cd engages is drilled in the second optical member 21Cc. The rear engaged hole 21 Cci is formed to have an almost same shape to suit the almost conical shape of the engaging portion 21 Ceb at the one end of the series-connecting member 21 Ce.

An engaged hole 21 Cdh with which the corresponding engaging portion 21 Ceb at the other end of the series-connecting member 21 Ce for series connection with the second optical member 21Cc engages is drilled in the third optical member 21 Cd. The engaged hole 21 Cdh is formed to have an almost same shape to suit the almost conical shape of the engaging portion 21 Ceb at the other end of the series-connecting member 2 1 Ce.

In the present embodiment as well, an incident light beam from a front surface of the first optical member 21 Cb may enter the second optical member 21Cc via a part outside an outermost perimeter of a diaphragm member 21f and become a stray light beam.

In order to suppress such a stray light beam, in the present embodiment, a part 32 (a region indicated by a grid pattern in Fig. 12) near an outer edge of the first optical member 21Cb is subjected to coloring with a light-shielding black color.

Note that the present embodiment is not limited to the method, and the same method as the third embodiment, i.e., a method of subjecting a part (31) outside the outermost perimeter of the diaphragm member 21f to coloring with the light-shielding black color may be used. Additionally, a method used in the present embodiment can also be applied to the third embodiment.

In the present embodiment as well, a light-shielding cylindrical member 21Bg in an almost cylindrical shape is provided to cover a whole exterior of an objective lens unit 21C, like the third embodiment. With the arrangement, entry of an unnecessary light beam from a side into the objective lens unit 21C is blocked, and an outer peripheral side of the plurality of optical members is fixedly held.

Other configurations are almost the same as the third embodiment described above.

The image pickup unit 24C according to the fourth embodiment with the above-described configuration is assembled in a procedure below.

One end of the series-connecting member 21 Ce is fit into each engaged hole 21 Cbh of the first optical member 21 Cb and is adhesively fixed.

The other end of each of the series-connecting members 21 Ce is fit into the corresponding front engaged hole 21 Cch of the second optical member 21Cc and is adhesively fixed. At the time, a rear surface of the first optical member 21 Cb and a front surface of the second optical member 21 Cc are connected in series with each other with the diaphragm member 21f sandwiched between the surfaces.

One end of the series-connecting member 21 Ce is fit into each rear engaged hole 21 Cci of the second optical member 21 Cc and is adhesively fixed.

The other end of each series-connecting member 21 Ce is fit into the corresponding engaged hole 21 Cdh of the third optical member 21 Cd and is adhesively fixed. At the time, a rear surface of the second optical member 21 Cc and a front surface of the third optical member 21 Cd are connected in series with each other.

The objective lens unit 21C, into which the plurality of optical members 21 Cb, 21 Cc, and 21 Cd are unitized by series connection and fixation, is formed in the above-described manner. The light-shielding cylindrical member 21Bg is arranged for the objective lens unit 21C to cover the whole exterior of the objective lens unit 21C.

The objective lens unit 21C is adhesively fixed to a predetermined position on a front side of an image pickup device 22. With the operation, the image pickup unit 24C according to the present embodiment is assembled.

As has been described above, according to the fourth embodiment, almost the same effects as the third embodiment can be achieved. Additionally, since each pair of adjacent ones of the optical members are individually connected in series with each other at the time of series connection and fixation of the plurality of optical members using the series-connecting members 21 Ce, positioning adjustment of each of the plurality of optical members can be performed more finely.

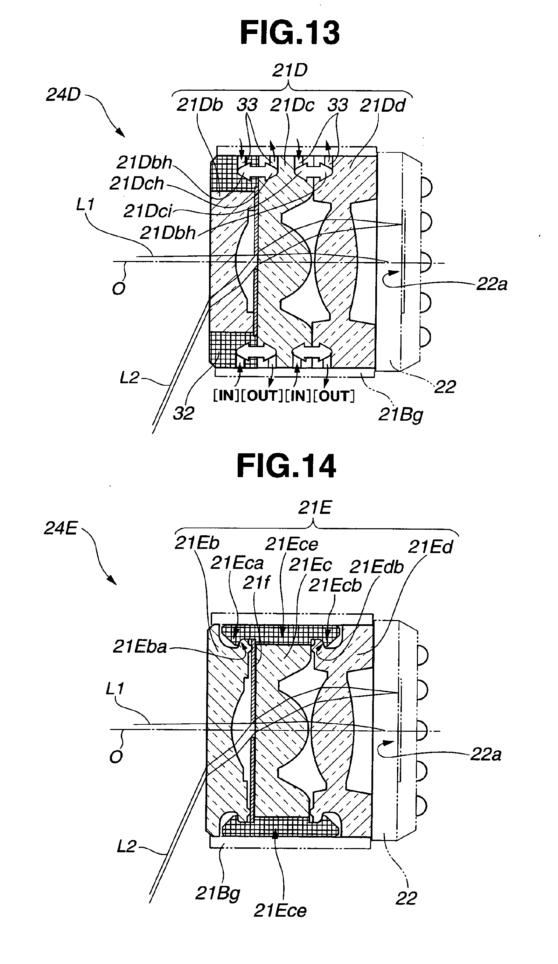

An image pickup unit according to a fifth embodiment of the present invention will be described below with reference to Fig. 13.

Fig. 13 is an enlarged sectional view showing, in an enlarged scale, the image pickup unit according to the fifth embodiment of the present invention.

As shown in Fig. 13, a basic configuration of an image pickup unit 24D according to the present embodiment is almost the same as the fourth embodiment described above and is slightly different in a configuration of a series-connecting portion for unitizing a plurality of optical members by connecting the plurality of optical members in series with each other.

That is, in the fourth embodiment, the plurality of optical members 21 Cb, 21 Cc, and 21 Cd are configured to be connected in series with each other using the series-connecting members 2 1 Ce.

On the other hand, in the image pickup unit 24D according to the present embodiment, three optical members 21 Cb, 21 Cc, and 21 Cd are series-connected and unitized by injecting a resin or the like into engaged holes formed in optical members 21Db, 21Dc, and 21Dd, instead of using the series-connecting members 21Ce.

That is, an engaged hole is formed in each of the plurality of optical members 21Db, 21Dc, and 21Dd according to the present embodiment. More specifically, the first optical member 21Db has an engaged hole 21Dbh formed on a rear side, the second optical member 21Dc has an engaged hole 21Dch formed on a front side and an engaged hole 21Dci formed on a rear side, and the third optical member 21Dd has an engaged hole 21 Ddh formed on a front side. The engaged holes (21Dbh, 21Dch, 21Dci, and 21Ddh) communicate with respective through holes 33 which have openings at outer peripheral surfaces of the optical members 21Db, 2 1 Dc, and 21Dd.

When the three optical members 21Db, 21Dc, and 21Dd are combined to be unitized such that optical axes almost coincide with each other at an optical axis O, cross-sectional shapes formed by the engaged holes (21 Dbh, 21Dch, 21Dci, and 21Ddh) are each almost the same as a cross-sectional shape (see Fig. 12) of each series-connecting member 21 Ce according to the fourth embodiment. That is, each cross-sectional shape is an elongated columnar shape with almost conical shapes at two ends.

Note that, as for the engaged holes to be formed in the optical members 21 Db, 21 Dc, and 21 Dd, the engaged holes of each type are arranged at three positions equally spaced in a circumferential direction.

Other configurations are almost the same as the fourth embodiment described above.

The image pickup unit 24D according to the fifth embodiment with the above-described configuration is assembled in a procedure below.

First, the three optical members 21Db, 21Dc, and 21Dd are arranged such that the optical axes almost coincide at the optical axis O and are fixed on, e.g., a jig to maintain the state.

In the state, a resin or the like is injected from a side indicated by an arrow [IN] in Fig. 13 into the through holes 33 formed in the optical member 21Db. A space formed by each pair of the engaged holes 21 Dbh and 21 Dch is filled with the resin or the like. When the injection is further continued, the resin or the like is forced out from a side indicated by an arrow [OUT] in Fig. 13. At the time, the injection is stopped.

The resin or the like is similarly injected from a side indicated by an arrow [IN] in Fig. 13 into the through holes 33 formed in the optical member 21Dc. A space formed by each pair of the engaged holes 21Dci and 21Ddh is filled with the resin or the like. When the injection is further continued, the resin or the like is forced out from a side indicated by an arrow [OUT] in Fig. 13. At the time, the injection is stopped.

When the injected resin or the like cures in the engaged holes, the three optical members 21Db, 21Dc, and 21Dd enter a state of being series-connected and fixed.

An objective lens unit 21D, into which the plurality of optical members 21Db, 21 Dc, and 21 Dd are unitized by series connection and fixation, is formed in the above-described manner. A light-shielding cylindrical member 21Bg (indicated by chain double-dashed lines in Fig. 13) is arranged for the objective lens unit 21D to cover a whole exterior of the objective lens unit 21D.

The objective lens unit 21D is then adhesively fixed to a predetermined position on a front side of an image pickup device 22. With the operation, the image pickup unit 24D according to the present embodiment is assembled.

As has been described above, according to the fifth embodiment, almost the same effects as the fourth embodiment can be achieved. Additionally, since the present embodiment can be configured without using a series-connecting member as a separate member to series-connect and fix the plurality of optical members, it is possible to contribute to a reduction in size and manufacturing cost by reducing component members, save labor required for an assembly work process, and perform efficient assembly.

An image pickup unit according to a sixth embodiment will be described below with reference to Fig. 14.

Fig. 14 is an enlarged sectional view showing, in an enlarged scale, the image pickup unit according to the sixth embodiment of the present invention.

As shown in Fig. 14, a basic configuration of an image pickup unit 24E according to the present embodiment is almost the same as the third embodiment described above. The present embodiment is different in that the image pickup unit 24E has a series-connecting portion 21Ece integrally formed near an outer edge of a second optical member 21Ec as a series-connecting portion replacing each series-connecting portion (series-connecting member 21Be), and engaged portions (21Eba and 21Edb) with which engaging portions 21Eca and 21Ecb of the series-connecting portion 2 1 Ece engage are formed in first and third optical members 21Eb and 21Ed, respectively.

More specifically, in the present embodiment, the second optical member 21 Ec of the plurality of optical members 21Eb, 21Ec, and 21Ed constituting an objective lens unit 21E is connected in series with the other optical members (21Eb and 21Ed) by engaging with the optical members and is formed to have the series-connecting portions 21Ece for unitizing the plurality of optical members.

Each series-connecting portion 21Ece is formed of the same member as the second optical member 21Ec integrally with the second optical member 21Ec near the outer edge of the second optical member 21 Ec. The series-connecting portion 21Ece is formed to extend in a direction parallel to an optical axis O, and a front distal end of the series-connecting portion 21Ece projects forward from a front surface of the second optical member 21 Ec and has the front engaging portion 21 Eca in a hooked shape at a most distal end portion. Similarly, a rear distal end of the series-connecting portion 21 Ece projects backward from a rear surface of the second optical member 21Ec and has the rear engaging portion 21Ecb in a hooked shape at a most distal end portion.

Note that each series-connecting portion 21Ece is subjected to coloring with a light-shielding black color in order to block, e.g., entry of an unnecessary light beam from an outside.

The first optical member 21 Eb is arranged in front of the second optical member 21Ec. In the case, the first optical member 21Eb and second optical member 21 Ec are positioned relative to each other such that optical axes of the first optical member 21 Eb and second optical member 21 Ec coincide with each other at the optical axis O shown in Fig. 14.

Each engaged portion 21 Eba, with which the front engaging portion 21 Eca of the corresponding series-connecting portion 21Ece engages, is formed near an outer edge of a surface opposed to the front surface of the second optical member 21 Ec in the first optical member 21 Eb. Engagement of the front engaging portions 21 Eca of the series-connecting portions 21 Ece with the engaged portions 21 Eba causes the first optical member 21 Eb to be connected in series with the second optical member 21Ec.

The third optical member 21Ed is arranged behind the second optical member 21Ec. In the case, the third optical member 21 Ed and second optical member 21Ec are positioned relative to each other such that optical axes of the third optical member 21Ed and second optical member 21Ec coincide with each other at the optical axis O shown in Fig. 14.

Each engaged portion 21 Edb, with which the rear engaging portion 21 Ecb of the corresponding series-connecting portion 21Ece engages, is formed near an outer edge of a surface opposed to the rear surface of the second optical member 21 Ec in the third optical member 21 Ed. Engagement of the rear engaging portions 21 Ecb of the series-connecting portions 21 Ece with the engaged portions 21 Edb causes the third optical member 21 Ed to be connected in series with the second optical member 21Ec.

The series-connecting portions 21 Ece formed integrally with the second optical member 21Ec are arranged at, e.g., three positions equally spaced in a circumferential direction near the outer edge of the objective lens unit 21B.

The engaged portions 21Eba and 21Edb are formed at corresponding parts of the first and third optical members 21Eb and 21Ed to correspond to the series-connecting portions 21 Ece.

Like the third, fourth, and fifth embodiments described above, a light-shielding cylindrical member 21Bg which covers a whole exterior of the objective lens unit 21E is arranged to function as a light-shielding member and a fixing frame.

Note that, in the present embodiment, the three optical members 21Eb, 21Ec, and 21 Ed are connected in series with each other at the time of formation of the objective lens unit 21E as a unit. Positioning of each optical member in a front-back direction (a direction parallel to the optical axis O) in the case is performed using abutment surfaces of the adjacent optical members. For the reason, vertical effect generating portions 21Ebe are formed, e.g., at a rear side surface of the first optical member 21Eb. Similarly, vertical effect generating portions 21Ede are formed at a front side surface of the third optical member 21 Ed.

Other configurations are almost the same as the first and second embodiments described above.

The image pickup unit 24E according to the sixth embodiment with the above-described configuration is assembled in a manner below.

First, the first optical member 21Eb is arranged in front of the second optical member 21Ec. At the time, the engaged portions 21 Eba of the first optical member 21 Eb are caused to engage with the front engaging portions 21 Eca of the series-connecting portions 21Ece of the second optical member 21Ec.

The third optical member 21 Ed is then arranged behind the second optical member 21Ec in a same manner. At the time, the engaged portions 21Edb of the third optical member 21Ed are caused to engage with the rear engaging portions 21 Ecb of the series-connecting portions 21 Ece of the second optical member 21 Ec.

The objective lens unit 21E, into which the plurality of optical members 21Eb, 21 Ec, and 21 Ed are unitized by series connection and fixation, is formed in the above-described manner. The light-shielding cylindrical member 21Bg (indicated by chain double-dashed lines in Fig. 14) is arranged for the objective lens unit 21E to cover the whole exterior of the objective lens unit 21E.

The objective lens unit 21E is then adhesively fixed to a predetermined position on a front side of an image pickup device 22. With the operation, the image pickup unit 24E according to the present embodiment is assembled.

As has been described above, according to the sixth embodiment, almost the same effects as the embodiments described above can be achieved. Additionally, the series-connecting portions 21 Ece, which series-connect and fix the plurality of optical members, are formed integrally with the second optical member 21 Ec, and the three optical members are series-connected and fixed by causing predetermined parts (the engaged portions 21Eba and 21Edb) of the plurality of optical members 21 Eb and 21 Ed arranged in front of and behind the second optical member 21 Ec to be engaged with the engaging portions 21Eca and 21Ecb of the series-connecting portions 21Ece. It is thus possible to reliably perform positioning and fixation of the plurality of optical members using a small number of component members and contribute to an improvement in assemblability.

Note that the present invention is not limited to the above-described embodiments, and various modifications and applications, of course, may be made without departing from scope of the invention.

The present application is filed claiming priority from Japanese Patent Application No. 2006-254819 filed in Japan on September 20,2006. A disclosure of the basic application is incorporated in the specification, claims, and drawings of the present application.

### Industrial Applicability

The present invention can be widely applied to a piece of electronic equipment such as a digital camera or cellular phone which is configured to have an image pickup device for picking up an electronic image, in addition to electronic endoscope apparatuses in medical and industrial fields.

## Claims

1. An image pickup unit comprising:
an objective lens unit composed of a plurality of optical members arranged in series with each other in a direction parallel to a single optical axis such that optical axes of the plurality of optical members coincide with each other and a series-connecting portion which unitizes the plurality of optical members; and
a photoelectric conversion image pickup device which receives an optical image formed by the objective lens unit and performs photoelectric conversion processing,
wherein the series-connecting portion includes a holding portion which holds one of the plurality of optical members, an extending portion which is provided to extend from one end of the holding portion in a direction parallel to the optical axis of the optical member held by the holding portion and is formed to be elastically deformable in a direction almost orthogonal to the optical axis, and an engaging portion which is provided at the extending portion and engages with, of the plurality of optical members, one different from the optical member held by the holding portion.

2. The image pickup unit according to claim 1, wherein the holding portion of the series-connecting portion and one of the plurality of optical members are formed integrally with each other.

3. The image pickup unit according to claim 1, wherein the holding portion of the series-connecting portion is composed of a cylindrical member provided to cover an outer edge of at least one of the plurality of optical members.

4. The image pickup unit according to claim 1 or 2, wherein
the engaging portion of the series-connecting portion is formed in a hooked shape, and
the engaging portion engages with an engaged portion formed at an outer edge of the different optical member.

5. The image pickup unit according to claim 1 or 2, wherein
the engaging portion of the series-connecting portion is composed of a male thread or a female thread, and
the engaging portion fits into or onto a female thread portion or a male thread portion formed at the outer edge of the different optical member.

6. The image pickup unit according to claim 1 or 2, wherein the series-connecting portion further includes a second extending portion which is provided to extend from the other end of the holding portion in the direction parallel to the optical axis of the optical member held by the holding portion and is formed to be elastically deformable in a direction almost orthogonal to the optical axis and a second engaging portion which is provided at the second extending portion and engages with, of the plurality of optical members, a third optical member other than the optical member held by the holding portion and the different optical member.

7. An image pickup unit comprising:
an objective lens unit composed of a plurality of optical members arranged in series with each other in a direction parallel to a single optical axis such that optical axes of the plurality of optical members coincide with each other and a series-connecting portion which unitizes the plurality of optical members; and
a photoelectric conversion device which receives an optical image formed by the objective lens unit and performs photoelectric conversion processing,
wherein the series-connecting portion includes an engaging portion which holds, of the plurality of optical members, a first optical member, a series-connecting member which extends from one end of the engaging portion in a direction parallel to the optical axis of the first optical member and is formed to be elastically deformable in a direction almost orthogonal to the optical axis, and an engaging portion which is provided at the series-connecting member and engages with, of the plurality of optical members, a second optical member.

8. An image pickup unit comprising:
a photoelectric conversion device which performs photoelectric conversion processing;
a first optical member;
a second optical member arranged in series with the first optical member such that optical axes of the first and second optical members almost coincide with each other;
a cylindrical portion which holds the first optical member;
an extending portion which extends from one end of the cylindrical portion in a direction parallel to the optical axes and is formed to be elastically deformable in a direction almost orthogonal to the optical axes; and
an engaging portion which is provided at a distal end of the extending portion and engages with the second optical member.

9. An endoscope composed of an image pickup unit according to any one of claims 1 to 8.
